# FASCICULE DE BREVET EUROPEEN

(11) **EP 0 753 503 B1**
(45) Date de publication et mention de la délivrance du brevet: **09.06.1999**
(21) Numéro de dépôt: 96401498.9
(22) Date de dépôt: 08.07.1996
(51) Int. Cl.: C07C 59/285, C07C 51/245, C07C 51/295

(54) **Procédé de fabrication de l'acide xylarique et utilisations de celui-ci**
Verfahren zur Herstellung von Xylarsäure und ihre Verwendung
Process for preparation of xylaric acid and use thereof

(30) Priorité: 11.07.1995 FR 9508363
(43) Date de publication de la demande: 15.01.1997
(73) Titulaire: Roquette Frères, 62136 Lestrem (FR)
(72) Inventeur: Fleche, Guy, 59190 Hazebrouck (FR)
(74) Mandataire: Boulinguiez, Didier

(56) Documents cités:
- WO-A-94/04650
- CH-B- 190 540
- DE-C- 620 248
- US-A- 2 338 114
- US-A- 4 833 230
- F. VENEMA ET AL.: "Platinium-catalyzed oxidation of aldopentoses to aldaric acids", JOURNAL OF MOLECULAR CATALYSIS, , , Vol. 77, no. , pages 75 à 85

## Description

La présente invention a pour objet un procédé de fabrication de l'acide xylarique.

De façon plus précise, elle a pour objet un procédé de fabrication de l'acide xylarique par dégradation oxydative alcaline de l'acide 5 cétogluconique.

L'acide xylarique, de formule semi-développée HOOC-(CHOH)3-COOH est un acide trihydroxydicarboxylique appartenant à la classe des acides trihydroxyglutariques, dont les utilisations pourraient être nombreuses s'il advenait qu'on puisse le produire en grosses quantités.

En tant qu'acide polyhydroxycarboxylique, il possède comme la plupart des acides de cette classe, des propriétés complexantes qui pourraient lui valoir une place de choix dans la formulation des compositions détergentes sans phosphates. A ce titre, la demande mondiale de brevet WO 94/04650 revendique pour ce type de composition détergente, l'utilisation d'acides polyhydroxydicarboxyliques ayant de 4 à 6 atomes de carbone et au moins 2 fonctions hydroxy par molécule. Sont cités parmi les acides remplissant ces conditions, les acides tartriques (4 atomes de carbone et 2 fonctions hydroxy) et les acides galactarique et glucarique (6 atomes de carbone et 4 fonctions hydroxy).

En tant qu'acide dicarboxylique, portant des fonctions acides aux extrémités d'une chaîne carbonée linéaire, l'acide xylarique peut entrer dans la composition de polymères du type polyhydroxypolyamides. Ces polymères sont constitués de motifs alternant régulièrement un motif d'acide aldarique et un motif d'une diamine primaire. Ils trouvent des applications dans les domaines du textile, des verres de sécurité, des colles, des émulsions aqueuses de peinture, des fibres, des matières plastiques, des cristaux liquides. A ce titre, le brevet américain US 4.833.230 décrit des polyhydroxypolyamides obtenus à partir d'acide glucarique, d'acide galactarique et d'acide xylarique.

En tant qu'acide trihydroxydicarboxylique, l'acide xylarique peut être réduit, par exemple à l'aide d'hydrures métalliques ou par hydrogénation catalytique, en le pentitol correspondant qui est le xylitol. Le xylitol est un polyalcool montrant un grand intérêt en tant qu'édulcorant.

Le xylitol est un produit existant à l'état naturel, presque aussi sucré que le sucre, mais qui possède sur le sucre, l'avantage de ne pas engendrer de caries dentaires.

Cependant, les procédés mis au point à ce jour pour obtenir l'acide xylarique ne donnent pas satisfaction. Ces procédés d'obtention de l'acide xylarique et plus généralement d'un acide aldarique, consistent principalement à oxyder l'aldose correspondant avec de l'acide nitrique (C.E. CANTRELL et coll. J. Org. Chem., 42, 3562 (1977)), plus rarement, à oxyder cet aldose à l'aide d'oxygène et d'un catalyseur métallique (F.R. VENEMA et coll. J. of Molecular Catalysis, 77 (1992) 75-85). Ces procédés donnent toutefois de mauvais rendements. Dans le cas de l'oxydation du xylose par l'acide nitrique, on obtient moins de 45 % d'acide xylarique et il est nécessaire d'utiliser des solvants organiques coûteux. A l'aide de catalyseurs à base de platine-alumine, la transformation, sans récupération du produit cristallisé, n'excède pas 58 %. Mais surtout, ces procédés d'oxydation souffrent de l'inconvénient d'utiliser le xylose comme matière première de la fabrication de l'acide xylarique. Le xylose est en effet un pentose d'obtention difficile qui n'est libéré qu'en petites quantités par l'hydrolyse dans des conditions drastiques de matières premières hémicellulosiques telles que le bois, la paille, les rafles de maïs.

Aucun de ces procédés utilisés pour obtenir de l'acide xylarique n'a jusqu'à présent fait preuve de son économie ou de son efficacité, confinant ainsi ce produit aux potentialités intéressantes dans un rôle plus proche des curiosités de laboratoire que ceux auxquels il pourrait prétendre de produit industriel ou de grand intermédiaire de synthèse.

Il existait donc un besoin de mettre au point un procédé performant d'obtention de l'acide xylarique, à partir de matières premières abondantes et peu coûteuses et c'est ce qu'est parvenue à faire la Demanderesse après avoir identifié ce besoin.

La présente invention a donc pour objet principal un procédé de fabrication de l'acide xylarique qui consiste à dégrader oxydativement l'acide 5 cétogluconique en milieu alcalin à l'aide d'oxygène ou d'un gaz en contenant.

Plus précisément, le procédé de fabrication de l'acide xylarique selon l'invention, repose sur le fait que la Demanderesse a découvert que le procédé de SPENGLER et PFANNENSTIEL (Brevet allemand n° 620.248) pouvait être appliqué avec succès à l'acide 5 cétogluconique pour fournir l'acide xylarique.

Ces inventeurs avaient remarqué que de l'air finement divisé au sein d'une solution alcaline d'un aldose ou d'un cétose dégradait celui-ci avec un bon rendement en l'acide aldonique correspondant de rang immédiatement inférieur d'une part et en acide formique d'autre part.

Conduite sur glucose, mannose ou fructose, cette dégradation oxydative alcaline se traduit surtout par l'obtention des acides arabinonique et formique, conduite sur le sorbose, comme indiqué par exemple dans la demande de brevet PCT 93/19030, elle amène surtout à la production des acides xylonique et formique.

Divers perfectionnements ont été apportés au procédé de SPENGLER et PFANNENSTIEL par exemple, H.E.J. HENDRIKS et collaborateurs, Carbohydrate Research, 214 (1991) 71-85 ont proposé, pour augmenter les rendements et la spécifité de la réaction d'oxydation des sucres réducteurs, d'utiliser comme catalyseur de dégradation oxydative, l'anthraquinone 2 sulfonate activé par le peroxyde d'hydrogène et d'effectuer la réaction avec de l'oxygène pur. Le brevet américain US 2.587.906 préconisait d'employer des catalyseurs constitués par le bleu de méthylène et ses analogues des séries thiazine, oxazine et phénazine pour oxyder des sucres réducteurs. Le brevet américain US 4.125.559 recommandait surtout, dans le même but, de travailler avec de l'oxygène pur sous des pressions pouvant aller jusqu'à 40 bars.

Le brevet US 2 338 114 (ISBELL) décrit un procédé de dégradation oxydative en milieu alcalin à l'aide d'oxygène, d'un acide hexuronique tel que l'acide galacturonique ou l'acide glucuronique pour donner l'acide arabinarique. Ce procédé ne pouvait en aucun cas être utilisé pour obtenir l'acide xylarique uniquement en utilisant comme acide glucuronique de départ l'acide iduronique ou l'acide guluronique qui sont tous les deux extrêmement rares.

Cependant, à la connaissance de la Demanderesse, ce type de dégradation oxydative alcaline n'a jamais été employé sur l'acide 5 cétogluconique qui à vrai dire, n'est ni un aldose, ni un cétose.

La présente invention a donc également pour objet un procédé de fabrication de l'acide xylarique consistant à dégrader oxydativement l'acide 5 cétogluconique en milieu alcalin à l'aide d'oxygène ou d'un gaz en contenant, caractérisé par le fait que la dégradation oxydative est effectuée en présence de catalyseurs et/ou sous pression.

Bien que l'acide 5 cétogluconique ait déjà été proposé comme matière première de la synthèse de l'acide tartrique, de la vitamine C, du 5 carboxy-D-gluconate ou des dérivés 5 amino de l'acide gluconique, à la connaissance de la Demanderesse, il n'a cependant jamais été proposé pour obtenir l'acide xylarique ni a fortiori comme intermédiaire dans la synthèse du xylitol. De la même manière, il ne semble pas que l'acide xylarique ait déjà été proposé spécifiquement comme un bon complexant du calcium.

L'acide 5 cétogluconique dont BOUTROUX a, pour la première fois, décrit l'obtention dès 1880, est très facilement obtenu à partir du glucose par fermentation aérobie de ce glucose à l'aide de souches diverses d'acetobacter ou de gluconobacter et ce dans un milieu contenant des nutriments apportés par exemple, par une liqueur de macération de maïs. Une telle fermentation est décrite notamment dans le brevet américain US 2.318.641.

La récupération et la purification du 5 cétogluconate de calcium en fin de fermentation se trouve grandement facilitée par le fait que ce sel de calcium de l'acide 5 cétogluconique obtenu sous la forme Ca (C6H8O7)2, 2 1/2 H2O est extrêmement peu soluble dans l'eau (environ 2g/l à 20°C) et qu'il peut ainsi être aisément débarrassé de ses contaminants principaux que sont le 2 cétogluconate de calcium ou le glucose non fermenté, par simple filtration.

L'acide libre peut être régénéré facilement à partir de son sel de calcium purifié par précipitation du sulfate ou de l'oxalate de calcium à l'aide d'acide sulfurique ou oxalique, ou transformé en un autre sel soluble de sodium ou de potassium par neutralisation subséquente avec une base idoine.

Pour obtenir l'acide xylarique selon le procédé de l'invention, on peut s'y prendre comme suit ou de manière équivalente :

Dans un réacteur pourvu de moyens d'agitation et d'aération ainsi que d'un système de régulation de la température, on met en solution ou en suspension dans de l'eau à une concentration de préférence comprise entre 50 et 500 g/l un sel d'acide 5 cétogluconique. On ajoute alors à cette solution ou suspension une base correspondant généralement au sel d'acide mis en oeuvre, en quantité telle qu'elle soit à même de constituer une réserve alcaline suffisante pour maintenir jusqu'au terme de la dégradation oxydative alcaline un pH supérieur à 12,5.

Cette base peut aussi être introduite progressivement dans le milieu réactionnel au fur et à mesure du déroulement de la réaction de dégradation oxydative en veillant à maintenir le pH du milieu réactionnel à une valeur supérieure à 12,5.

A l'inverse, on peut aussi préparer une solution de la base et y introduire progressivement le sel de l'acide 5 cétogluconique.

Pour être efficace et s'effectuer avec de bons rendements, il est également préférable, comme l'ont démontré SPENGLER et PFANNENSTIEL, que la réaction de dégradation oxydative alcaline se déroule en présence d'air très finement divisé dans le milieu. L'air peut bien entendu être enrichi en oxygène ou remplacé par de l'oxygène. De même on peut travailler à des pressions supérieures à la pression atmosphérique, on peut également effectuer cette réaction en présence de catalyseurs. Ceci permet d'augmenter la sélectivité et le rendement de cette réaction. D'une manière générale, on parvient à une division assez fine des bulles de gaz en agitant violemment le milieu réactionnel à l'aide d'hélices ou de turbines.

La réaction de dégradation alcaline oxydative de l'acide 5 cétogluconique qui se traduit essentiellement par la formation d'une mole d'acide xylarique et d'une mole d'acide formique par mole d'acide 5 cétogluconique mise en oeuvre, s'effectue de préférence en maintenant la température du milieu réactionnel entre 20 et 60° C. On préfère toutefois travailler en maintenant cette température entre 40 et 50° C.

Lorsqu'on opère cette dégradation alcaline oxydative en présence d'un catalyseur, on préfère employer un mélange équimoléculaire d'anthraquinone 2 sulfonate et de peroxyde d'hydrogène en une quantité comprise généralement entre 0,1 g et 5 g de catalyseur par mole d'acide 5 cétogluconique mise en oeuvre.

Dans les conditions décrites ci-dessus la réaction de dégradation alcaline oxydative est d'ordinaire menée à terme en environ 3 à 24 heures.

Ce terme peut être commodément évalué par la mesure du pouvoir réducteur, vis à vis d'un réactif cupro-tartrosodique par exemple, du milieu réactionnel.

On préfère arrêter la réaction quand le milieu réactionnel ne montre plus qu'un pouvoir réducteur inférieur à 5 %, de préférence inférieur à 2 % du pouvoir réducteur initial.

A ce stade, un tel milieu brut réactionnel peut, après en avoir enlevé les éventuels catalyseurs, constituer, de préférence après séchage, une excellente base complexante pour produits lessiviels. Les impuretés présentes et qui sont essentiellement des petites quantités de sels d'acides gluconique, glycérique, tartronique, malique, tartrique, érythronique... et de plus grosses quantités d'acide formique possèdent en effet elles aussi, des propriétés complexantes non négligeables.

Cependant, lorsque l'on désire obtenir l'acide xylarique à une pureté permettant son utilisation dans la synthèse des polyhydroxypolyamides ou du xylitol, on préfère, lorsque l'on a effectué la réaction de dégradation oxydative alcaline de l'acide 5 cétogluconique sous la forme d'un de ses sels très solubles, notamment de sodium ou de potassium, précipiter l'acide xylarique sous la forme de son sel de calcium en ajoutant au milieu réactionnel une quantité stoechiométrique ou en léger excès, de chaux, de chlorure de calcium ou de carbonate de calcium par exemple.

La Société Demanderesse a en effet remarqué que le xylarate de calcium était extrêmement peu soluble dans l'eau contrairement aux sels de calcium des autres acides très souvent présents, même en faible quantité après la réaction de dégradation oxydative alcaline et contrairement à l'acide formique toujours présent. La présente invention a donc aussi pour objet un procédé de fabrication de l'acide xylarique obtenu par dégradation alcaline oxydative de l'acide 5 cétogluconique, caractérisé par le fait qu'il consiste à fabriquer et à isoler comme produit intermédiaire le xylarate de calcium.

Pour obtenir un précipité de xylarate de calcium plus aisément filtrable, on peut effectuer cette opération à une température élevée, pouvant aller jusqu'au point d'ébullition du milieu réactionnel.

Après refroidissement du milieu réactionnel, on isole le xylarate de calcium précipité par simple filtration. On peut éliminer les sels solubles de calcium retenus par le gâteau de filtration par un lavage de celui-ci. Un tel lavage permet d'éliminer les impuretés qui se sont formées au cours de l'étape de dégradation alcaline oxydative du 5 cétogluconate. Il permet surtout d'éliminer le formiate qui est produit en même temps que le xylarate lors de cette étape.

On peut ensuite libérer l'acide xylarique de son sel de calcium à l'aide d'un acide tel que l'acide oxalique ou l'acide sulfurique. Ici encore un réchauffage vers 70°C du milieu réactionnel, suivi d'un refroidissement, permettra d'obtenir des cristaux d'oxalate ou de sulfate de calcium mieux formés et plus facilement filtrables.

On recommande aussi lors de cette étape de libération de l'acide xylarique, de n'employer l'acide sulfurique ou l'acide oxalique qu'en quantité légèrement inférieure à la stoechiométrie, se réservant d'enlever le calcium encore présent par déminéralisation sur une résine cationique. Cette manière de faire permet d'éviter de retrouver dans la solution d'acide xylarique, trop d'acide sulfurique ou oxalique dont il serait difficile de se débarrasser par la suite.

Une simple concentration, à une matière sèche supérieure à 50 %, de la solution d'acide xylarique issue de la résine cationique permet l'obtention de cristaux de cet acide sans avoir recours à un quelconque solvant organique. On préfère effectuer cette concentration à faible température et donc dans un appareillage fonctionnant sous vide. On obtient des cristaux mieux formés et beaucoup plus réguliers lorsque l'on ensemence avec des cristaux broyés d'acide xylarique, la solution concentrée légèrement sursaturée en cet acide. De même, il est préférable, après cette évaporation, de refroidir lentement et sous agitation douce, la bouillie de cristaux obtenue afin d'en faciliter le fractionnement ultérieur.

Les cristaux d'acide xylarique, d'une pureté chimique supérieure à 98 % sont ensuite facilement isolés de la bouillie de cristaux obtenue précédemment par simple essorage sous l'action de la force centrifuge. Il peut également être avantageux, lorsque l'on désire accroître encore la pureté des cristaux, de procéder à une petite pulvérisation d'eau sur le gâteau de cristaux soumis à l'essorage, de façon à en éliminer de façon pratiquement complète, les eaux-mères qui leur ont donné naissance.

Ces eaux-mères peuvent être soumises à une nouvelle concentration qui fournit alors un deuxième jet de cristaux d'acide xylarique. Elles peuvent être également recyclées avec le contenu du réacteur immédiatement après la réaction de dégradation oxydative alcaline de l'acide 5 cétogluconique.

L'acide xylarique ainsi obtenu peut être utilisé en tant que matière première dans la fabrication de polyhydroxypolyamides.

Il peut également et ce, de préférence après une lactonisation, subir une hydrogénation catalytique à l'aide d'un catalyseur au ruthénium par exemple pour obtenir le xylitol.

Les exemples qui suivent ont pour objet d'illustrer et de mieux faire comprendre l'invention. Ils ne sauraient être limitatifs en particulier pour ce qui concerne la nature du sel d'acide 5 cétogluconique mis en oeuvre pour la réaction de dégradation alcaline oxydative ou celle des acides employés pour libérer les acides 5 cétogluconique ou xylarique.

### Exemples :

### Exemple 1 : Dégradation oxydative alcaline de l'acide 5 cétogluconique

On traite à l'acide oxalique en quantité stoechiométrique, une suspension à 10 % de 5 cétogluconate de calcium en agitant et en portant à 70° C durant une heure cette suspension.

Après refroidissement, on filtre le tout sur une précouche de terre filtrante pour éliminer l'oxalate de calcium.

On obtient une solution d'acide 5 cétogluconique, limpide et légèrement orangée que l'on percole au travers d'une colonne de résines cationiques fortes pour éliminer un éventuel excès de calcium.

Après avoir neutralisé à pH 7,0, cette solution d'acide 5 cétogluconique à l'aide de lessive de soude, on concentre celle-ci sous vide à une matière sèche de 50 % correspondant à une densité de 1,294 g/cm3.

Dans un fermenteur à cuve de verre de 15 litres de volume utile, on introduit 7 litres d'eau et 787,2 g d'une lessive de soude à 50 % soit 9,84 moles de NaOH, que l'on porte à 40 ° C puis on aère cette solution durant 30 minutes.

On ajoute alors dans cette solution, 12 grammes d'anthraquinone 2 sulfonate de sodium soit 3,7 . 10-2 mole et 4 cm3 d'eau oxygénée à 30 % soit 4 . 10-2 mole de H2O2. On introduit dès lors de façon continue dans ce réacteur 1374 cm3 de la solution de 5 cétogluconate de sodium obtenue précédemment au débit approximatif de 8ml/mn durant 170 minutes, soit un total de 4,1 moles de 5 cétogluconate de sodium.

Durant ce temps on règle l'agitation et l'aération du milieu réactionnel de façon à maintenir une coloration rose de ce milieu. Cette coloration indique que l'on ne se trouve ni en excès, ni en défaut d'oxygène dissous dans le fermenteur qui sert de réacteur.

Après 170 minutes, tout en maintenant l'aération et l'agitation, on augmente la température à 50° C puis après 255 minutes on la porte à 60° C. On arrête la réaction après 375 minutes.

La teneur du milieu réactionnel en sucres réducteurs, exprimés en "équivalent glucose", telle que mesurée à la liqueur cupro-tartro-sodique, s'établit alors à 1,15 g par litre de milieu réactionnel indiquant par là même un pouvoir réducteur équivalent à 10 g de sucres réducteurs présents dans le milieu réactionnel, soit environ 0,057 mole "d'équivalent glucose", soit encore environ 1,4 % du pouvoir réducteur initial : 0,057 x 100/4,1.

Ce milieu réactionnel, de couleur rouge sombre lorsque toute aération est arrêtée, est ensuite traité sur une colonne de charbon actif granulaire, après en avoir rectifié le pH à 5,5 par addition de 25 ml d'acide sulfurique de densité 1,98, de manière à en enlever l'anthraquinone 2 sulfonate de sodium puis est concentré à une matière sèche de 20,4 %.

L'analyse effectuée sur ce produit a donné les résultats suivants :

| | |
|---|---|
| acide xylarique | 77,8 g/l |
| acide formique | 27,7 g/l |
| acide glycolique | 11,6 g/l |
| acide glycérique | 1,7 g/l |
| acide tartrique | 11,9 g/l |
| acide malique | 4,4 g/l |
| acide tartronique | 7 g/l |
| sulfate disodique | 50 g/l |

Après séchage par atomisation, un tel produit brut, contenant les sels de sodium des acides xylarique et formique mais aussi en quantités moindres, ceux des acides glycolique, glycérique, tartrique, malique, tartronique... peut former une excellente base complexante du calcium et ses propriétés peuvent être exploitées notamment dans les formules lessivielles à la base des compositions détergentes sans phosphates ou à teneur réduite en phosphates.

A ce stade, le rendement molaire en xylarate de sodium, atteint 51,4 % par rapport au 5 cétogluconate mis en oeuvre. Il pourrait être facilement amélioré en procédant à la dégradation oxydative alcaline de l'acide 5 cétogluconique à l'aide d'oxygène et/ou en travaillant sous pression.

### Exemple 2 : Purification de l'acide xylarique

On traite à l'aide de carbonate de calcium, à raison d'une demi mole de carbonate de calcium par mole de sodium présente, le milieu réactionnel brut et concentré à 20,4 % obtenu à l'exemple 1. Ce traitement est effectué sous agitation à une température voisine de 100° C. Après refroidissement, on observe un abondant précipité blanc constitué pour l'essentiel de xylarate de calcium très peu soluble.

On filtre ce précipité que l'on lave à l'aide d'un peu d'eau froide puis on le remet en suspension dans de l'eau de manière à obtenir un lait d'une matière sèche de 20 % environ. On porte alors ce lait à 70° C puis on y ajoute sous agitation de l'acide oxalique en quantité suffisante pour précipiter environ 95 % du calcium présent sous forme d'oxalate de calcium. On laisse ensuite refroidir lentement et sous agitation douce ce lait, puis on élimine l'oxalate par filtration, ce qui fournit une solution brute d'acide xylarique dont on enlève le calcium résiduel par percolation sur une résine cationique forte.

On concentre alors sous vide cette solution jusqu'à une matière sèche de 50 %, puis on l'ensemence à l'aide de cristaux broyés d'acide xylarique avant de poursuivre cette évaporation jusqu'à l'obtention d'une masse épaisse de cristaux.

L'essorage de ces cristaux après lavage sur l'essoreuse puis séchage dans un appareil à lit fluidisé avec de l'air à 50° C fournit de très beaux cristaux d'acide xylarique d'une richesse de 99,2 %.

### Exemple 3 : Utilisation de l'acide xylarique pour la synthèse du xylitol

On a fait fondre des cristaux d'acide xylarique à l'air durant 30 minutes à la température de 145° C.

Le liquide obtenu s'est pris en bloc lors de son refroidissement et ce bloc a été pulvérisé pour fournir une poudre blanche répondant à l'analyse suivante :

| | |
|---|---|
| acide xylarique | 11 % |
| lactone | 78 % |
| dimères | 11 % |
| eau par méthode de KARL FISCHER | 2,5 % |

On a remis cette poudre en solution dans de l'eau, à 40 % de matière sèche puis on a soumis cette solution à une pression d'hydrogène de 80 bars, à 130° C en présence d'un catalyseur constitué de 2 % de ruthénium supporté par du charbon actif, à raison de 0,2 % de ruthénium par rapport à la matière sèche mise en oeuvre.

L'analyse du produit résultant de cette hydrogénation a montré la présence de 3,7 % de xylitol après 5 heures de réaction.

### Exemple 4 : Utilisation de l'acide xylarique comme complexant du calcium

On a mesuré le pouvoir séquestrant du xylarate obtenu à l'exemple 1 à pH 10 et à la température de 40 ° C en présence d'acide borique et on l'a comparé au pouvoir séquestrant du citrate de soude et du tripolyphosphate de soude.

Les résultats sont exprimés en milligrammes de calcium complexé par gramme de produit sec

| | |
|---|---|
| xylarate | 58 |
| citrate | 68 |
| tripolyphosphate | 155 |

On constate donc que le produit brut de l'exemple 1, riche en xylarate, peut prétendre en raison de son bon pouvoir séquestrant, trouver un emploi dans les compositions lessivielles.

## Revendications

1. Procédé de fabrication de l'acide xylarique, caractérisé par le fait qu'il consiste à dégrader oxydativement l'acide 5-cétogluconique ou un sel de l'acide 5-cétogluconique , en milieu alcalin, à l'aide d'oxygène ou d'un gaz en contenant.

2. Procédé selon la revendication 1, caractérisé par le fait que le milieu alcalin résulte de l'addition d'une base, de préférence un sel basique de l'acide 5-cétogluconique.

3. Procédé selon la revendication 1 ou 2, caractérisé par le fait que l'acide 5-cétogluconique ou le sel de l'acide 5-cétogluconique est dégradé oxydativement à un pH supérieur à 12,5.

4. Procédé selon l'une quelconque des revendications 1 à 3, caractérisé par le fait que l'oxygène ou le gaz contenant de l'oxygène est finement divisé dans le milieu réactionnel.

5. Procédé de fabrication de l'acide xylarique selon l'une quelconque des revendications 1 à 4, caractérisé par le fait que la dégradation oxydative alcaline est effectuée en présence de catalyseurs.

6. Procédé selon la revendication 5, caractérisé par le fait que le catalyseur est un mélange équimolaire d'anthraquinone-2-sulfonate et de peroxyde d'hydrogène

7. Procédé selon l'une quelconque des revendications 1 à 6, caractérisé par le fait que la dégradation oxydative est conduite à une température allant de 20 à 60°C.

8. Procédé de fabrication de l'acide xylarique selon l'une quelconque des revendications 1 à 7, caractérisé par le fait que la dégradation oxydative alcaline est effectuée sous pression.

9. Procédé selon l'une quelconque des revendications 1 à 8, caractérisé par le fait que la dégradation oxydative est conduite jusqu'à ce que le milieu réactionnel présente un pouvoir réducteur de moins de 5%, de préférence de moins de 2% du pouvoir réducteur initial.

10. Procédé selon l'une quelconque des revendications 1 à 5, caractérisé par le fait que l'acide xylarique est retiré du milieu réactionnel par addition dans celui-ci de chaux, de chlorure de calcium ou de carbonate de calcium dans des quantités stoechiométriques ou en léger excès par rapport à la quantité d'acide xylarique formé, par récupération du xylarate de calcium précipité ainsi formé et traitement de celui-ci par un acide choisi dans le groupe comprenant l'acide oxalique ou l'acide sulfurique.

## Claims

1. Process for the manufacture of xylaric acid, characterized in that it consists in oxydatively degrading 5-ketogluconic acid or a salt of 5-ketogluconic acid with oxygen or a gas containing it, in an alkaline medium.

2. Process according to claim 1, characterized in that the alkaline medium is provided by the addition of a base, preferably a basic salt of 5-ketogluconic acid.

3. Process according to claim 1 or 2, characterized in that 5-ketogluconic acid or the salt of 5-ketogluconic acid is oxidatively degraded at a pH of above 12.5.

4. Process according to any one of claims 1 to 3, characterized in that the oxygen or the gaz containing oxygen is finely divided in the reaction medium.

5. Process for the manufacture of xylaric acid according to any one of claims 1 to 4, characterized in that the alkaline oxidative degradation is carried out in the presence of catalysts.

6. Process according to claim 5, characterized in that the catalyst is an equimolar mixture of anthraquinone-2-sulphonate and hydrogen peroxide.

7. Process according to any one of claims 1 to 6, characterized in that the oxidative degradation is carried out at a temperature ranging from 20 to 60°C.

8. Process for the manufacture of xylaric acid according to any one of claims 1 to 7, characterized in that the alkaline oxidative degradation is carried out under pressure.

9. Process according to any one of claims 1 to 8, characterized in that the oxidative degradation is carried out until the reaction medium shows a reductive power of less than 5%, preferably less than 2% of the initial reductive power.

10. Process according to any one of claims 1 to 5, characterized in that xylaric acid is recovered from the reaction medium by adding thereto lime, calcium chloride or calcium carbonate in stoichiometric amounts or in slight excess with respect to the amount of xylaric acid formed, recovering the thus formed precipated calcium xylarate and treating it with an acid selected from the group comprising oxalic acid or sulphuric acid.

## Patentansprüche

1. Verfahren zur Herstellung von Xylarsäure, dadurch gekennzeichnet, daß es darin besteht, die 5-Ketogluconsäure oder ein Salz der 5-Ketogluconsäure im alkalischen Medium mit Hilfe von Sauerstoff oder einem Sauerstoff enthaltenden Gas oxidativ abzubauen.

2. Verfahren nach Anspruch 1, dadurch gekennzeichnet, daß das alkalische Medium aus der Zugabe einer Base, vorzugsweise eines basischen Salzes der 5-Ketogluconsäure resultiert.

3. Verfahren nach Anspruch 1 oder 2, dadurch gekennzeichnet, daß die 5-Ketogluconsäure oder das Salz der 5-Ketogluconsäure bei einem pH-Wert von höher als 12,5 oxidativ abgebaut werden.

4. Verfahren nach irgendeinem der Ansprüche 1 bis 3, dadurch gekennzeichnet, daß der Sauerstoff oder das den Sauerstoff enthaltende Gas in dem Reaktionsmedium fein verteilt werden.

5. Verfahren zur Herstellung von Xylarsäure nach irgendeinem der Ansprüche 1 bis 4, dadurch gekennzeichnet, daß der alkalische, oxidative Abbau in Anwesenheit von Katalysatoren durchgeführt wird.

6. Verfahren nach Anspruch 5, dadurch gekennzeichnet, daß der Katalysator eine äquimolare Mischung von 2-Anthrachinon-sulfonat und Wasserstoffperoxid ist.

7. Verfahren nach irgendeinem der Ansprüche 1 bis 6, dadurch gekennzeichnet, daß der oxidative Abbau bei einer Temperatur von 20 °C bis 60 °C durchgeführt wird.

8. Verfahren zur Herstellung von Xylarsäure nach irgendeinem der Ansprüche 1 bis 7, dadurch gekennzeichnet, daß der alkalische, oxidative Abbau unter Druck durchgeführt wird.

9. Verfahren nach irgendeinem der Ansprüche 1 bis 8, dadurch gekennzeichnet, daß der oxidative Abbau solange durchgeführt wird, bis das Reaktionsmedium ein Reduktionsvermögen von weniger als 5 %, vorzugsweise von weniger als 2 % des anfänglichen Reduktionsvermögens aufweist.

10. Verfahren nach irgendeinem der Ansprüche 1 bis 5, dadurch gekennzeichnet, daß die Xylarsäure aus dem Reaktionsmedium durch Zugabe von Kalk, Calciumchlorid oder Calciumcarbonat zu dem Reaktionsmedium in stöchiometrischen Mengen oder einem leichten Überschuß in bezug auf die gebildete Menge von Xylarsäure, durch Gewinnung des auf diese Weise gebildeten und ausgefällten Calciumxylarates und dessen Behandlung mit einer Säure, gewählt aus der Oxalsäure oder Schwefelsäure umfassenden Gruppe, abgezogen wird.
